(19) Europäisches Patentamt
European Patent Office
Office européen des brevets

(11) Veröffentlichungsnummer: **0 256 097**
**B1**

(12) # EUROPÄISCHE PATENTSCHRIFT

(45) Veröffentlichungstag der Patentschrift: **01.08.90**

(21) Anmeldenummer: **87901266.4**

(22) Anmeldetag: **16.12.86**

(86) Internationale Anmeldenummer:
**PCT/EP86/00754**

(87) Internationale Veröffentlichungsnummer:
**WO 87/04426 30.07.87 Gazette 87/17**

(60) **Teilanmeldung 89119488.8 eingereicht am 16/12/86.**

(51) Int. Cl.$^5$: **C 07 C 43/188,**
C 07 C 43/215, C 07 C 255/50,
C 07 C 43/115, C 07 C 43/21,
C 07 D 239/26,
C 07 D 319/06, C 09 K 19/30,
C 09 K 19/20, C 09 K 19/18,
C 09 K 19/34

(54) **Vinylen-Verbindungen und flüssigkristalline Phase.**

(30) Priorität: **20.01.86 DE 3601452**

(43) Veröffentlichungstag der Anmeldung:
**24.02.88 Patentblatt 88/08**

(45) Bekanntmachung des Hinweises auf die Patenterteilung:
**01.08.90 Patentblatt 90/31**

(84) Benannte Vertragsstaaten:
**AT BE CH DE GB LI NL**

(56) Entgegenhaltungen:
**EP-A-0 122 389**
**EP-A-0 136 501**
**EP-A-0 168 683**
**Chemical Abstracts, Band 102, Nr. 6, 11. Februar 1985, (Columbus, Ohio, US), C. Aquilera et al.: "Thermotropic liquid crystalline bimesogenic molecules with highly flexible oligosiloxane spacer", siehe Seite 560, Zusammenfassung Nr. 54271g, Polym. Bull (Berlin) 1984, 12(5), 383-8**
**Chemical Abstracts, Band 107, Nr. 2, 13. Julie 1987, (Columbus, Ohio, Us), siehe Seite 555, Zusammenfassung 15685d, & JP-A-61257935**
**Die Akte enthält technische Angaben, die nach dem Eingang der Anmeldung eingereicht wurden und die nicht in dieser Patentschrift enthalten sind.**

(73) Patentinhaber: **MERCK PATENT GESELLSCHAFT MIT BESCHRÄNKTER HAFTUNG**
**Frankfurter Strasse 250 Postfach 4119**
**D-6100 Darmstadt (DE)**

(72) Erfinder: **WÄCHTLER, Andreas**
**Goethestr. 34**
**D-6103 Griesheim (DE)**
Erfinder: **KRAUSE, Joachim**
**Samuel-Morse-Str. 14**
**D-6110 Dieburg (DE)**
Erfinder: **EIDENSCHINK, Rudolf**
**Konrad Adenauer Strasse 1**
**D-6115 Münster (DE)**
Erfinder: **SCHEUBLE, Bernhard**
**Bluff 100 100-1, Yamate-cho Naka-ku, Yokohama-shi**
**D-6109 Mühltal 1 (JP)**
Erfinder: **HITTICH, Reinhard**
**Am Kirchberg 11**
**D-6101 Modautal 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 256 097 B1

**Beschreibung**

Die Erfindung betrifft neue Vinylen-Verbindungen der Formel I

$$R^1—(A^1—Z^1)_m—(A^2—Z^2)_n—(A^3)_o—O—(CH_2)_p—CH=CH—R^2 \qquad I$$

worin

$R^1$ Alkyl mit 1 bis 12 C-Atomen, worin auch eine oder zwei nicht benachbarte $CH_2$-Gruppen durch —O—, —O—CO—, —CO—O—, —CO— und/oder —CH=CH— ersetzt sein können, F, Cl, Br oder CN,

$R^2$ H oder Alkyl mit 1 bis 8 C-Atomen,

$A^1$, $A^2$ und $A^3$ jeweils unabhängig voneinander unsubstituiertes oder durch ein oder zwei F- und/oder Cl-Atome und/oder $CH_3$-Gruppen und/oder CN-Gruppen substituiertes 1,4-Phenylen, worin auch eine oder zwei CH-Gruppen durch N-Atome ersetzt sein können, unsubstituiertes oder durch ein oder zwei F- und/oder Cl-Atome und/oder $CH_3$-Gruppen und/oder CN-Gruppen substituiertes 1,4-Cyclohexylen, worin auch eine oder zwei nicht benachbarte $CH_2$-Gruppen durch O-Atome ersetzt sein können, 1,3-Dithian-2,5-diyl, 1,4-Bicyclo(2,2,2)octylen, Naphthalin-2,6-diyl, 1,2,3,4-Tetrahydronaphthalin-2,6-diyl, 1,2,3,4-Tetrahydronaphthalin-2,6-diyl oder trans-Dekahydronaphthalin-2,6-diyl,

$Z^1$ und $Z^2$ jeweils unabhängig voneinander —CO—O, —O—CO—, O—$CH_2$—, $CH_2$—O, —$CH_2CH_2$—, —CH=N—, —N=CH—, —C≡C— oder eine Einfachbindung,

m und o 1 oder 2,

n 0 oder 1, und

p 1 oder 2

bedeuten, wobei für m = 2 und/oder o = 2 die beiden Gruppen $A^1$ bzw. $A^3$ gleich oder voneinander verschieden sein können, wobei für die Vertragstaaten CH, DE und GB die Maßgabe gilt daß falls,

n = 0, m = 1 und eine der Gruppen $A^1$ und $A^3$ eine 1,4-Phenylengruppe bedeutet, $Z^1$ nur —CO—O—, $OCH_2$—, —$CH_2$—O—, —$CH_2CH_2$— oder —C≡C— bedeutet, und daß

die Verbindungen

trans-4-Pentylcyclohexancarbonsäure-p-allyloxyphenylester

trans-4-(4-Pentenyl)cyclohexancarbonsäure-p-allyloxyphenylester

4-Allyloxy-1-[2-(trans-4-pentylcyclohexyl)-ethyl]-benzol

4-(2E-Butenyloxy)-1-[2-(trans-4-propylcyclohexyl)-ethyl]-benzol

4-(3-Butenyloxy)-1-[2-(trans-4-propylcyclohexyl)-ethyl]-benzol

4-(3-Butenyloxy)-1-[2-(trans-4-pentylcyclohexyl)-ethyl]-benzol

4-Allyloxy-2-fluor-1-[2-(trans-4-pentylcyclohexyl)-ethyl]-benzol

4-(3-Butenyloxy)-2-fluor-1-[2-(trans-4-pentylcyclohexyl)-ethyl]-benzol

ausgenommen sind.

Der Einfachheit halber bedeuten im folgenden Phe eine 1,4-Phenylengruppe, Cy eine 1,4-Cyclohexylengruppe, Dio eine 1,3-Dioxan-2,5-diylgruppe, Bi eine Bicyclo-(2,2,2)-octylengruppe und Pyr eine Pyrimidin-2,5-diylgruppe, wobei diese Gruppen, insbesondere die 1,4-Phenylengruppe und die 1,4-Cyclohexylgruppe, unsubstituiert oder substituiert sein können.

Die Verbindungen der Formel I können als Komponenten flüssigkristalliner Phasen verwendet werden, insbesondere für Displays, die auf dem Prinzip der verdrillten Zelle, dem Guest-Host-Effekt, dem Effekt der Deformation aufgerichteter Phasen oder dem Effekt der dynamischen Streuung beruhen.

Aus der EP—A2—0 136 501 sind ungesättigte Ether der Formel $R^1—A^1—A^2—O—C_nH_{2n-1}$ worin $A^1$ und $A^2$ jeweils unabhängig voneinander 1,4-Phenylen-und 1,4-Cyclohexylengruppen bedeuten, als Zwischenprodukte zur Herstellung der entsprechenden Trialkanoyloxysilane bekannt.

Ähnliche Verbindungen sind in der EP—A2—0 122 389 beschrieben, diese weise jedoch im Gegensatz zu den erfindungsgemäßen Verbindungen eine Alkenylgruppe anstelle einer Alkenyloxygruppe auf, deren Einführung durch die Wittig-Reaktion bei der Herstellung im Produktionsmaßstab große Schwierigkeiten bereitet.

Die aus der nicht ververöffentlichten EP—A2—O 168 683 bekannten Alkenyloxy-Verbindungen sind gemäß der Maßgabe b) für die Vertragsstaaten CH, DE und GB ausgenommen.

C. Aquilera und L. Bernal beschreiben in Polymer Bulletin 12, 383—388 (1984) thermotrope flüssigkristalline Oligosiloxane, welche aus p-Alkoxyphenyl p-allyloxybenzoaten hergestellt werden.

Der Erfindung lag die Aufgabe zugrunde, neue stabile Flüssigkristalline oder mesogene Verbindungen aufzufinden, die als Komponenten flüssigkristalliner Dielektrika geeignet sind.

Es wurde gefunden, daß die Verbindungen der Formel I flüssigkristalline Mesophasen in einem überraschend großen Temperaturbereich bilden und als Komponenten flüssigkristalliner Dielektrika vorzüglich geeignet sind. Insbesondere sind mit ihrer Hilfe stabile flüssigkristalline Phasen mit breitem Mesophasenbereich, mit guter Löslichkeit gegenüber dichroitischen Farbstoffen und/oder polaren Zusätzen, vergleichsweise niedriger Viskosität, teilweise besonders niedriger optischer Anisotropie und günstigen elastischen Eigenschaften herstellbar.

Mit der Bereitstellung der Verbindungen der Formel I wird außerdem ganz allgemein die Palette der flüssigkristallinen Substanzen, die sich unter verschiedenen anwendungstechnischen Gesichtspunkten zur Herstellung nematischer Gemische eignen, erheblich verbreitert.

Die Verbindungen der Formel I besitzen einen breiten Anwendungsbereich. In Abhängigkeit von der Auswahl der Substituenten können diese Verbindungen als Basismaterialien dienen, aus denen flüssigkristalline Phasen zum überwiegenden Teil zusammengesetzt sind; es können aber auch Verbindungen der Formel I flüssigkristallinen Basismaterialien aus anderen Verbindungsklassen zugesetzt werden, um beispielsweise die dielektrische und/oder optische Anisotropie eines solchen Dielektrikums zu senken. Die Verbindungen der Formel I eignen sich ferner als Zwischenprodukte zur Herstellung anderer Substanzen, die sich beispielsweise als Bestandteile flüssigkristalliner Phasen verwenden lassen.

Die Verbindungen der Formel I sind in reinem Zustand farblos und bilden flüssigkristalline Mesophasen in einem für die elektrooptische Verwendung günstig belegenen Temperaturbereich. Chemisch, thermisch und gegen Licht sind sie sehr stabil.

Gegenstand der Erfindung sind somit die Verbindungen der Formel I sowie die Verwendung der Verbindungen der Formel I als Komponenten flüssigkristalliner Phasen. Gegenstand der Erfindung sind ferner flüssigkristalline Phasen mit einem Gehalt an mindestens einer Verbindung der Formel I sowie elektrooptische Azeigeelemente, die als Dielektrika derartige flüssigkristalline Phasen enthalten.

Vor- und nachstehend haben $R^1$, $R^2$, $A^1$, $A^2$, $A^3$, $Z^1$, $Z^2$, m, n und o die angegebene Bedetung und Q bedeutet $-O-(CH_2)_p$, worin p 1 oder 2 ist, sofern nich ausdrücklich etwas anderes vermerkt ist.

Die Verbindungen der Formel I umfassen dementsprechend Verbindungen der Teilformel Ia (mit zwei Ringen), Ib und Ic (mit jeweils drei Ringen), Id, Ie und If (mit jeweils vier Ringen) sowie Ig (mit fünf Ringen):

$R^1-A^1-Z^1-A^3-Q-CH=CH-R^2$ Ia

$R^1-A^1-Z^1-A^2-Z^2-A^3-Q-CH=CH-R^2$ Ib

$R^1-A^1-A^1-Z^1-A^3-Q-CH=CH-R^2$ Ic

$R^1-A^1-A^1-Z^1-A^2-Z^2-A^3-Q-CH=CH-R^2$ Id

$R^1-A^1-Z^1-A^2-Z^2-A^3-A^3-Q-CH=CH-R^2$ Ie

$R^1-A^1-A^1-Z^1-A^3-A^3-Q-CH=CH-R^2$ If

$R^1-A^1-A^1-Z^1-A^2-Z^2-A^3-A^3-Q-CH=CH-R^2$ Ig

Die bevorzugten Verbindungen der Teilformel Ia umfassen solche der Teilformeln Iaa bis Iaj:

$R^1$-Phe-$Z^1$-Phe-Q-CH=CH-$R^2$ Iaa

$R^1$-Cy-$Z^1$-Phe-Q-CH=CH-$R^2$ Iab

$R^1$-Dio-$Z^1$-Phe-Q-CH=CH-$R^2$ Iaa

$R^1$-Pyr-$Z^1$-Phe-Q-CH=CH-$R^2$ Iad

$R^1$-Bi-$Z^1$-Phe-Q-CH=CH-$R^2$ Iae

$R^1$-Bi-$Z^1$-Cy-Q-CH=CH-$R^2$ Iaf

$R^1$-Cy-$Z^1$-Cy-Q-CH=CH-$R^2$ Iag

$R^1$-Dio-$Z^1$-Cy-Q-CH=CH-$R^2$ Iah

$R^1$-Pyr-$Z^1$-Cy-Q-CH=CH-$R^2$ Iai

$R^1$-Dio-$Z^1$-Pyr-Q-CH=CH-$R^2$ Iaj

Darunter sind diejenigen der Formeln Iaa, Iag und Iah besonders bevorzugt.

Von den Verbindungen der Teilformeln Ib, Ic, Id, Ie und If sind diejenigen der Teilformeln II bis I 40 besonders bevorzugt:

$R^1$-Phe-$Z^1$-Phe-$Z^2$-Phe-Q-CH=CH-$R^2$ I1

$R^1$-Phe-$Z^1$-Cy-$Z^2$-Phe-Q-CH=CH-$R^2$ I2

$R^1$-Cy-$Z^1$-Phe-$Z^2$-Phe-Q-CH=CH-$R^2$ I3

$R^1$-Cy-Phe-$Z^2$-Phe-Q-CH=CH-$R^2$ I4

$R^1$-Phe-$Z^1$-Phe-$Z^2$-Cy-Q-CH=CH-$R^2$ I5

$R^1$-Phe-$Z^1$-Cy-$Z^2$-Cy-Q-CH=CH-$R^2$ I6

$R^1$-Cy-$Z^1$-Phe-$Z^2$-Cy-Q-CH=CH-$R^2$ I7

$R^1$-Cy-Phe-$Z^2$-Cy-Q-CH=CH-$R^2$ I8

$R^1$-Cy-$Z^1$-Cy-$Z^2$-Phe-Q-CH=CH-$R^2$ I9

$R^1$-Cy-$Z^1$-Cy-$Z^2$-Cy-Q-CH=CH-$R^2$ I10

$R^1$-Dio-$Z^1$-Phe-$Z^2$-Phe-Q-CH=CH-$R^2$ I11

$R^1$-Dio-Phe-Phe-Q-CH=CH-$R^2$ I12

$R^1$-Phe-$Z^1$-Dio-$Z^2$-Phe-Q-CH=CH-$R^2$ I13

$R^1$-Phe-$Z^1$-Phe-$Z^2$-Dio-Q-CH=CH-$R^2$ I14

$R^1$-Cy-$Z^1$-Phe-$Z^2$-Dio-Q-CH=CH-$R^2$ I15

$R^1$-Dio-$Z^1$-Cy-$Z^2$-Phe-Q-CH=CH-$R^2$ I16

$R^1$-Dio-Cy-Phe-Q-CH=CH-$R^2$ I17

$R^1$-Dio-$Z^1$-Phe-$Z^2$-Cy-Q-CH=CH-$R^2$ I18

$R^1$-Pyr-$Z^1$-Phe-$Z^2$-Phe-Q-CH=CH-$R^2$ I19

$R^1$-Cy-$Z^1$-Pyr-$Z^2$-Phe-Q-CH=CH-$R^2$ I20

$R^1$-Dio-$Z^1$-Pyr-$Z^2$-Phe-Q-CH=CH-$R^2$ I21

$R^1$-Cy-$Z^1$-Phe-$Z^2$-Pyr-Q-CH=CH-$R^2$ I22

$R^1$-Dio-$Z^1$-Phe-$Z^2$-Pyr-Q-CH=CH-$R^2$ I23

$R^1$-Dio-$Z^1$-Cy-$Z^2$-Cy-Q-CH=CH-$R^2$ I24

$R^1$-Cy-$Z^1$-Dio-$Z^2$-Cy-Q-CH=CH-$R^2$ I25

$R^1$-Cy-$Z^1$-Cy-$Z^2$-Dio-Q-CH=CH-$R^2$ I26

$R^1$-Dio-$Z^1$-Cy-$Z^2$-Dio-Q-CH=CH-$R^2$ I27

$R^1$-Cy-Phe-Phe-$Z^2$-Cy-Q-CH=CH-$R^2$ I28

$R^1$-Cy-Phe-Phe-$Z^2$-Phe-Q-CH=CH-$R^2$ I29

$R^1$-Dio-Phe-Phe-$Z^2$-Cy-Q-CH=CH-$R^2$ I30

$R^1$-Pyr-Phe-Phe-$Z^2$-Cy-Q-CH=CH-$R^2$ I31

$R^1$-Cy-Phe-$Z^1$-Phe-$Z^2$-Cy-Q-CH=CH-$R^2$ I32

$R^1$-Cy-Phe-$Z^1$-Cy-$Z^2$-Cy-Q-CH=CH-$R^2$ I33

$R^1$-Cy-Phe-$Z^1$-Cy-$Z^2$-Phe-Q-CH=CH-$R^2$ I34

$R^1$-Cy-Phe-$Z^2$-Phe-Cy-Q-CH=CH-$R^2$ I35

$R^1$-Cy-Phe-$Z^2$-Phe-Phe-Q-CH=CH-$R^2$ I36

$R^1$-Dio-Phe-$Z^2$-Phe-Phe-Q-CH=CH-$R^2$ I37

$R^1$-Dio-Phe-$Z^2$-Pyr-Phe-Q-CH=CH-$R^2$ I38

$R^1$-Dio-Phe-$Z^2$-Cy-Phe-Q-CH=CH-$R^2$ I39

$R^1$-Dio-Phe-$Z^2$-Ph-Cy-Q-CH=CH-$R^2$ I40

In den Verbindungen der vor- und nachstehenden Formeln bedeutet $R^1$ vorzugsweise Alkyl, ferner Alkoxy oder eine andere Oxaalkylgruppe, F oder CN.

$A^1$, $A^2$ und $A^3$ sind bevorzugt Cy, DiO oder Phe, ferner bevorzugt Pyr; bevorzugt enthält die Verbindung der Formel I nicht mehr als einen der Reste Dio, Bi oder Pyr. $A^3$ is vorzugsweise Cy.

Besonders bevorzugt sind Verbindungen der Formel I, worin $A^1$, $A^2$ und $A^3$ jeweils unabhängig voneinander Cy oder Phe bedeuten.

Bevorzugte Verbindungen der Formel I enthalten unsubstituierte trans-1,4-Cyclohexylengruppen.

$Z^1$ und $Z^2$ sind bevorzugt Einfachbindungen oder $—CH_2CH_2—$, in zweiter Linie bevorzugt —CO—O— oder —O—CO—.

Besonders bevorzugt sind Verbindungen der Formel I, worin $—(A^2—Z^2)_n—(A^3)_o—$ eine Gruppe der Formel

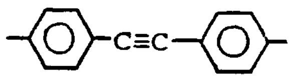

bedeutet.

Q ist vorzugsweise $—OCH_2—$ Q ist vorzugsweise direkt mit einer trans-1,4-Cyclohexylengruppe verknüpft.

Falls $R^1$ einen Alkylrest bedeutet, worin auch eine (Alkoxy bzw. Oxaalkyl) oder zwei (Alkoxyalkoxy bzw. Dioxaalkyl) nicht benachbarte $CH_2$-Gruppen durch O-Atome und/oder —CH=CH– ersetzt sein können, so können sie geradkettig oder verzweigt sein. Vorzugsweise sind sie geradkettig, haben 2, 3, 4, 5, 6 oder 7 C-Atome und bedeuten demnach bevorzugt Ethyl, Propyl, Butyl, Pentyl, Hexyl, Heptyl, Ethoxy, Propoxy, Butoxy, Pentoxy, Hexoxy, Heptoxy, 2-Oxapropyl (= Methoxymethyl), 2- (= Ethoxymethyl) oder 3-Oxabutyl (= 2-Methoxyethyl), 2-, 3- oder 4-Oxapentyl, 2-, 3-, 4- oder 5-Oxahexyl, 2-, 3-, 4-, 5- oder 6-Oxaheptyl, trans-1-Propenyl, trans-3-Propenyl, trans-1-Butenyl, trans-3-Butenyl, trans-1-Pentenyl, trans-3-Pentyl, trans-1-Hexenyl, trans-3-Hexenyl, trans-1-Heptenyl, trans-3-Heptenyl, ferner Methyl, Octyl, Nonyl, Decyl, Methoxy, Octoxy, Nonoxy, Decoxy, 2-, 3-, 4-, 5-, 6- oder 7-Oxactyl, 2-, 3-, 4-, 5-, 6-, 7- oder 8-Oxanonyl, 2-, 3-, 4-, 5-, 6-, 7-, 8- oder 9-Oxadecyl, 1,3-Dioxabutyl (= Methoxymethyl), 1,3-, 1,4- oder 2,4-Dioxapentyl, 1,3-, 1,4-, 1,5-, 2,4-, 2,5 oder 3,5-Dioxahexyl, 1,3-, 1,4-, 1,5-, 1,6-, 2,4-, 2,5-, 2,6-, 3,5-, 3,6- oder 4,6-Dioxaheptyl, trans-1-Octenyl, trans-3-Octenyl, trans-1-Nonenyl, trans-3-Nonenyl, trans-1-Decenyl oder trans-3-Decenyl.

$R^2$ ist vorzugsweise H oder geradkettiges Alkyl mit 1 bis 8 C-Atomen und bedeutet demnach bevorzugt H, Methyl, Ethyl, Propyl, Butyl, Pentyl, Hexyl, Heptyl oder Octyl. Besonders bevorzugte Bedeutungen für $R^2$ sind H, Methyl, Ethyl und Propyl.

Verbindungen der Formel I mit verzweigten Flügelgruppen $R^1$ bzw. $R^2$ können gelegentlich wegen einer besseren Löslichkeit in den üblichen flüssigkristallinen Basismaterialien von Bedeutung sein, insbesondere aber als chirale Dotierstoffe, wenn sie optisch aktiv sind. Verzweigte Gruppen dieser Art enthalten in der Regel nicht mehr als eien Kettenverzweigung. Bevorzugte verzweigte Reste $R^1$ und $R^2$ sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy, 2-Oxa-3-methylbutyl, 3-Oxa-4-methylpentyl.

Falls die Verbindungen der Formel I ein asymmetrisches C-Atom enthalten, umfaßt die Formel I Racemate und auch optisch aktive Enantiomeren und Enantiomerengemische.

Unter den Verbindungen der Formeln I sowie Ia bis Ig, Iaa bis Iaj und II bis I40 sind diejenigen bevorzugt, in denen mindestens einer der darin enthaltenen Reste eine der angegebenen bevorzugten Bedeutungen hat. Besonders bevorzugte kleinere Gruppen von Verbindungen sind diejenigen der Formel I 41 bis I63:

| | |
|---|---|
| $R^1$-Cy-Cy-Q-CH=CH-$R^2$ | I41 |
| $R^1$-Cy-Cy-Cy-Q-CH=CH-$R^2$ | I42 |
| $R^1$-Cy-Phe-Q-CH=CH-$R^2$ | I43 |
| $R^1$-Pyr-Phe-Q-CH=CH-$R^2$ | I44 |
| $R^1$-Phe-Phe-Q-CH=CH-$R^2$ | I45 |
| $R^1$-Cy-Phe-Cy-Q-CH=CH-$R^2$ | I46 |
| $R^1$-Dio-Phe-Q-CH=CH-$R^2$ | I47 |
| $R^1$-Cy-Phe-Phe-Cy-Q-CH=CH-$R^2$ | I48 |
| $R^1$-Cy-Phe-Phe-Q-CH=CH-$R^2$ | I49 |
| $R^1$-Phe-Phe-Cy-Q-CH=CH-$R^2$ | I50 |
| $R^1$-Phe-OCO-Phe-Q-CH=CH-$R^2$ | I51 |
| $R^1$-Phe-COO-Phe-Q-CH=CH-$R^2$ | I52 |
| $R^1$-Cy-COO-Phe-Q-CH=CH-$R^2$ | I53 |
| $R^1$-Phe-OCO-Cy-Q-CH=CH-$R^2$ | I54 |

$R^1$-Cy-COO-Cy-Q-CH=CH-$R^2$ I55

$R^1$-Cy-OCO-Cy-Q-CH=CH-$R^2$ I56

$R^1$-Cy-Pyr-Phe-Q-CH=CH-$R^2$ I57

$R^1$-Cy-$CH_2CH_2$-Cy-Q-CH=CH-$R^2$ I58

$R^1$-Phe-Cy-Q-CH=CH-$R^2$ I59

$R^1$-Phe-Pyr-Q-CH=CH-$R^2$ I60

$R^1$-Phe-Pyr-Phe-Q-CH=CH-$R^2$ I61

$R^1$-Phe-Pyr-Cy-Q-CH=CH-$R^2$ I62

$R^1$-Phe-Dio-Q-CH=CH-$R^2$ I63

Unter den Verbindungen der Formeln I41 bis I63 sind diejenigen bevorzugt, in denen $R^1$ Alkyl oder CN und/oder Cy oder Phe unsubstituiertes 1,4-Cyclohexylen oder 1,4-Phenylen bedeutet.

Unter den Verbindungen der Formel I, die eine oder mehrere der Gruppen Cy und/oder Dio enthalten, sind die in 1,4- bzw. 2,5-Stellung trans-substituierten Isomeren bevorzugt. Trägt die 1,4-Cyclohexylengruppe in 1- und/oder 4-Stellung einen oder zwei weitere Substituenten, so sind diese bevorzugt in axialer Position.

Diejenigen der vorstehend genannten Formeln, die eine oder mehrere Gruppen DiO, Pyr und/oder 1,3-Dithian-2,5-diyl enthalten, umschließen jeweils die beiden möglichen 2,5-Stellungsisomeren.

Besonders bevorzugt sind Verbindungen der Formel Iaa, worin $Z^1$ —C≡C— und Q —O—$CH_2$— oder —O—$CH_2$—$CH_2$— bedeutet, d.h. solche der Formel Iaa1

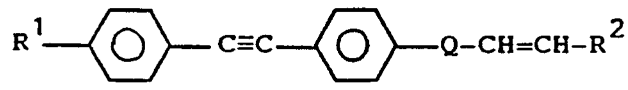 Iaa1.

$R^1$ und $R^2$ bedeuten hier vorzugsweise n-Alkyl mit jeweils 2 bis 10, insbesondere 2 bis 7, C-Atomen.

Die Verbindungen der Formel I werden nach an sich bekannten Methoden hergestellt, wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die gekannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

So können die Verbindungen der Formel I, worin Q —O—$(CH_2)_p$— bedeutet, hergestellt werden, in dem man eine Verbindung der Formel II,

$$R^1—(A^1)_m—Z^1—(A^2—Z^2)_n—(A^3)_o—OH \qquad II$$

worin $R^1$, $A^1$, $A^2$, $A^3$, $Z^1$, $Z^2$, m, n und o die angegebene Bedeutung haben, mit einer entsprechenden Alkenylverbindung verethert.

Hierbei wird die Hydroxyverbindung der Formel II zweckmäßig zunächst in ein entsprechendes Metallderivat, z.B. durch Behandeln mit NaOH, KOH, $Na_2CO_3$ oder $K_2CO_3$ in das entsprechende Alkalimetallalkoholat oder Alkalimetallphenolat übergeführt wird. Dieses kann dann mit dem entsprechenden Alkenylhalogenid, -sulfonat oder Dialkenylsulfat umgesetzt werden, zweckmäßig in einem inerten Lösungsmittel wie Aceton, DMF oder Dimethylsulfoxid oder auch einem Überschuß an wäßriger oder wäßrig-alkoholischer NaOH oder KOH bei Temperaturen zwischen etwa 20°C und 100°C.

Die Verbindungen der Formeln II sind entweder bekannt, oder sie können ohne Schwierigkeiten nach an sich bekannten Methoden in Analogie zu bekannten Verfahren hergestellt werden.

Bei der Synthese der Hydroxyverbindungen der Formel II können im Prinzip alle Methoden angewendet werden, die für die Verbindungen bekannt sind, die an Stelle der OH-Gruppe andere Substituenten tragen. Der Fachmann kann die erforderlichen Synthesevarianten nach Routinemethoden ableiten.

Die erfindungsgemäßen flüssigkristallinen Phasen bestehen aus 2 bis 25, vorzugsweise 3 bis 15 Komponenten, darunter mindestens einer Verbindung der Formel I. Die anderen Bestandteile werden vorzugsweise ausgewählt aus den nematischen oder nematogenen Substanzen, insbesondere den bekannten Substanzen, aus den Klassen der Azoxybenzole, Benzylidenaniline, Biphenyle, Terphenyle, Phenyl- oder Cyclohexylbenzoate, Cyclohexan-carbonsäurephenyl- oder -cyclohexyl-ester, Phenylcyclohexane, Cyclohexylbiphenyle, Cyclohexylcyclohexane, Cyclohexylnaphthaline, 1,4-Biscyclohexylbenzole, 4,4'-Bis-cyclohexylbiphenyle, Phenyl- oder Cyclohexylpyrimidine, Phenyl- oder

Cyclohexyldioxane, gegebenenfalls halogenierten Stilbene, Benzylphenylether, Tolane und substituierten Zimtsäuren.

Die wichtigsten als Bestandteile derartiger flüssigkristalliner Phasen in Frage kommenden Verbindungen lassen sich durch die Formel VI charakterisieren,

$$R^3—L—G—E—R^4 \qquad VI$$

worin L und E je ein carbo- oder heterocyclisches Ringsystem aus der aus 1,4-disubstituierten Benzol- und Cyclohexanringen, 4,4'-disubstituierten Biphenyl-, Phenylcyclohexan- und Cyclohexylcyclohexansystemen, 2,5-disubstituierten Pyrimidin- und 1,3-Dioxanringen, 2,6-disubstituiertem Naphthalin, Di- und Tetrahydronaphthalin, Chinazolin und Tetrahydrochinazolin gebildeten Gruppe

| | | |
|---|---|---|
| G | —CH=CH— | —N(O)=N— |
| | —CH=CY | —CH=N(O)— |
| | —C≡C— | $—CH_2—CH_2—$ |
| | —CO—O— | $—CH_2—O—$ |
| | —CO—S— | $—CH_2—S—$ |
| | —CH=N— | —COO—Phe—COO |

oder eine C—C-Einfachbindung, Y Halogen, vorzugsweise Chlor, oder —CN, und $R^3$ und $R^4$ Alkyl, Alkoxy, Alkanoyloxy oder Alkoxycarbonyloxy mit bis zu 18, vorzugsweise bis zu 8 Kohlenstoffatomen, oder einer dieser Reste auch CN, NC, $NO_2$, $CF_3$, F, Cl oder Br bedeuten.

Bei den meisten dieser Verbindungen sind $R^3$ und $R^4$ voneinander verschieden, wobei einer dieser Reste meist meist eine Alkyl- oder Alkoxygruppe ist. Aber auch andere Varianten der vorgesehen Substituenten sind gebräuchlich. Viele solcher Substanzen oder auch Gemische davon sind im Handel erhältlich. Alle diese Substanzen sind nach an sich bekannten Methoden herstellbar.

Die erfindungsgemäßen flüssigkristallinen Phasen enthalten etwa 0,1 bis 99, vorzugsweise 1 bis 30%, einer oder mehrerer Verbindungen der Formel I, 1 bzw. 2.

Die Herstellung der erfindungsgemäßen flüssigkristallinen Phasen erfolgt in an sich üblicher Weise. In der Regel werden die Komponenten ineinander gelöst, zweckmäßib bei erhöhte Temperatur.

Durch geeignete Zustände können die flüssigkristallinen Phasen nach der Erfindung so modifiziert werden, daß sie in allen bisher bekannt gewordenen Arten von Flüssigkristallanzeigeelementen verwendet werden können.

Derartige Zusätze sind dem Fachmann bekannt und in der Literatur ausführlich beschrieben. Beispielsweise können Leitsalze, vorzugsweise Ethyl-dimethyl-dodecylammonium-4-hexyloxybenzoat, Tetrabutylammonium-tetraphenylboranat oder Komplexsalze von Kronenethern (vgl. z.B. I. Haller et al., Mol. Cryst. Liq. Cryst. Band *24*, Seiten 249—258 (1973)) zur Verbesserung der Leitfähigkeit, dichroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden. Derartige Substanzen sind z.B. in den DE—OS 22 09 127, 22 40 864, 23 21 632, 23 38 281, 24 50 088, 26 37 430, 28 53 728 und 29 02 177 beschrieben.

Die folgenden Beispiele sollen die Erfindung erläutern, ohne sie zu begrenzen. F. = Schmelzpunkt, K. = Klärpunkt. Vor- und nachstehend bedeuten Prozentangaben Gewichtsprozent; alle Temperaturen sind in Grad Celsius angegeben. "Übliche Aufarbeitung" bedeutet: man gibt Wasser hinzu, extrahiert mit Methylenchlorid, trennt, ab, trocknet die organische Phase ein und reinigt das Produkt durch Kristallisation, Destillation und/oder Chromatographie.

### Beispiel 1

Zu einer Alkoholatlösung aus 225 g trans-4-(trans-4-n-Propylcyclohexyl)-cyclohexanol und 5,8 g Natrium gibt man 29,6 g Allylbromid und kocht unter Rühren 5 Stunden. Nach üblicher Aufarbeitung erhält man trans-4-(trans-4-n-Propylcyclohexyl)-1-allyloxycyclohexan, F. 18,8°, K. 35,8°.

Analog werden hergestellt:

trans-4-(trans-4-Methylcyclohexyl)-1-allyloxycyclohexan
trans-4-(trans-4-Ethylcyclohexyl)-1-allyloxycyclohexan
trans-4-(trans-4-Butylcyclohexyl)-1-allyloxycyclohexan, F. 6°, K. 38°
trans-4-(trans-4-Pentylcyclohexyl)-1-allyloxycyclohexan, F. 26°, K. 54°
trans-4-(trans-4-Hexylcyclohexyl)-1-allyloxycyclohexan
trans-4-(trans-4-Heptylcyclohexyl)-1-allyloxycyclohexan
trans-4-(trans-4-Methylcyclohexyl)-1-E-2-buten-1-yloxycyclohexan
trans-4-(trans-4-Ethylcyclohexyl)-1-E-2-buten-1-yloxycyclohexan

trans-4-(trans-4-Propylcyclohexyl)-1-E-2-buten-1-yloxycyclohexan
trans-4-(trans-4-Butylcyclohexyl)-1-E-2-buten-1-yloxycyclohexan
trans-4-(trans-4-Pentylcyclohexyl)-1-E-2-buten-1-yloxycyclohexan
trans-4-(trans-4-Hexylcyclohexyl)-1-E-2-buten-1-yloxycyclohexan
trans-4-(trans-4-Heptylcyclohexyl)-1-E-2-buten-1-yloxycyclohexan
trans-4-(trans-4-Methylcyclohexyl)-1-E-2-penten-1-yloxycyclohexan
trans-4-(trans-4-Ethylcyclohexyl)-1-E-2-penten-1-yloxycyclohexan
trans-4-(trans-4-Propylcyclohexyl)-1-E-2-penten-1-yloxycyclohexan
trans-4-(trans-4-Butylcyclohexyl)-1-E-2-penten-1-yloxycyclohexan
trans-4-(trans-4-Pentylcyclohexyl)-1-E-2-penten-1-yloxycyclohexan
trans-4-(trans-4-Hexylcyclohexyl)-1-E-2-penten-1-yloxycyclohexan
trans-4-(trans-4-Heptylcyclohexyl)-1-E-2-penten-1-yloxycyclohexan
trans-4-(trans-4-Methylcyclohexyl)-1-E-2-hexen-1-yloxycyclohexan
trans-4-(trans-4-Ethylcyclohexyl)-1-E-2-hexen-1-yloxycyclohexan
trans-4-(trans-4-Propylcyclohexyl)-1-E-2-hexen-1-yloxycyclohexan
trans-4-(trans-4-Butylcyclohexyl)-1-E-2-hexen-1-yloxycyclohexan
trans-4-(trans-4-Pentylcyclohexyl)-1-E-2-hexen-1-yloxycyclohexan
trans-4-(trans-4-Hexylcyclohexyl)-1-E-2-hexen-1-yloxycyclohexan
trans-4-(trans-4-Heptylcyclohexyl)-1-E-2-hexen-1-yloxycyclohexan

## Beispiel 2

Eine Lösung von 16 g p-(trans-4-n-Propylcyclohexyl)-phenol in 20 ml Methylenchlorid wird unter Rühren in eine Natriumalkohollösung aus 2,1 g Na in 80 ml Methanol getropft. Anschließend werden 9,2 g Allylbromid zugegeben. Das Gemisch wird 5 Stunden gekocht und wie üblich aufgearbeitet. Man erhält trans-1-p-Allyloxyphenyl-4-propylcyclohhexan, F. 25°.

Analog werden hergestellt:

trans-1-p-Alloxyphenyl-4-ethylcyclohexan
trans-1-p-Allyloxyphenyl-4-butylcyclohexan
trans-1-p-Allyloxyphenyl-4-pentylcyclohexan, F. 32°, K. 41°
trans-1-p-Allyloxyphenyl-4-heptylcyclohexan
trans-1-p-(E-2-Penten-1-yloxyphenyl-4-ethylcyclohexan
trans-1-p-(E-2-Penten-1-yloxyphenyl)-4-proylcyclohexan
trans-1-p-(E-2-Penten-1-yloxyphenyl)-4-butylcyclohexan
trans-1-p-(E-2-Penten-1-yloxyphenyl)-4-pentylcyclohexan
trans-1-p-(E-2-Buten-1-yloxyphenyl)-4-ethylcyclohexan
trans-1-p-(E-2-Buten-1-yloxyphenyl)-4-propylcyclohexan F. 42°, K. 57°
trans-1-p-(E-2-Buten-1-yloxyphenyl)-4-butylcyclohexan
trans-1-p-(E-2-Buten-1-yloxyphenyl)-4-pentylcyclohexan, F. 32°, K. 67°.
2-p-Allyloxyphenyl-5-butylpyrimidin, F. 56°
2-p-Allyloxyphenyl-5-pentylpyrimidin
2-p-Allyloxyphenyl-5-hexylpyrimidin
2-p-Allyloxyphenyl-5-heptylpyrimidin, F. 43°, K. 50°
2-p-Allyloxyphenyl-5-octylpyrimidin
2-p-Allyloxyphenyl-5-nonylpyrimidin
2-p-Allyloxyphenyl-5-decylpyrimidin
2-p-(3-Butenyloxy)-phenyl-5-butylpyrimidin, F. 37°
2-p-(4-Pentenyloxy)-phenyl-5-butylpyrimidin, F. 36°
2-p-Allyloxyphenyl-5-pentyl-1,3-dioxan, F. 42°
1-(trans-4-Ethylcyclohexyl)-2-(p-allyloxyphenyl)-ethan
1-(trans-4-Propylcyclohexyl)-2-(p-allyloxyphenyl)-ethan, F. 24°, K. 25°
1-(trans-4-Butylcyclohexyl)-2-(p-allyloxyphenyl)-ethan
1-(trans-4-Pentylcyclohexyl)-2-(p-allyloxyphenyl)-ethan, F. 15°, K. 41°
1-(trans-4-Heptylcyclohexyl)-2-(p-allyloxyphenyl)-ethan
1-(trans-4-Propylcyclohexyl)-2-(p-E-2-buten-1-yloxyphenyl)-ethan. F. 44°, K. 52°
1-(trans-4-Propylcyclohexyl)-2-(3-butenyloxyphenyl)-ethan, F. 6°, K. 16°
1-(trans-4-Propylcyclohexyl)-2-(4-pentenyloxyphenyl)-ethan, F. 32°, K. 30°.

## Beispiel 3

Ein Gemisch aus 41,2 p-(E-2-Penten-1-yloxy)-benzoesäure (herstellbar durch Alkylierung von p-Hydroxybenzoesäure mit (E)-2-Penten-1-ylbromid (erhältlich aus (E)-2-Pentenol (A. E. Gastaminzu et al., J. Org. Chem. *49*, 3859 (1984) durch Bromierung mit $PPh_3/Br_2$ (analog R. Machinek, W. Lüttke, Synthesis *1975*, 225)) und 23,8 g p-Hydroxybenzonitril in 250 ml $CH_2Cl_2$ wird unter Feuchtigkeitsausschluß nach W. Steglich et al., Angew. Chem. *90*, 556 (1978) bei 5°C mit 2,4 g DMAP versetzt. Dazu tropft man dann unter Rühren und Eiskühlung eine Lösung von 45,3 DCC in 50 ml $CH_2Cl_2$. Die Temperatur darf dabei +5°C nicht überschreiten. Anschließend wird das Reaktionsgemisch 5 Stunden bei Raumtemperatur gerührt. Dann

wird der ausgefallene Harnstoff abgesaugt und das Filtrat mit kalter, verdünnter HCl gewaschen. Die organische Phase wird anschließend mit Wasser gewaschen, mit $Na_2SO_4$ getrocknet. Nach dem Abdampfen des Lösungsmittels wird der Rückstand durch Kristallisation gereinigt. Man erhält p-(E-2-Penten-1-yloxy)-benzoesäure-p-cyanophenylester.

Analog wurden hergestellt:
p-(E-2-Hepten-1-yloxy)-benzoesäure-p-cyanophenylester
p-(E-2-Hexen-1-yloxy)-benzoesäure-p-cyanophenylester
p-(E-2-Buten-1-yloxy)-benzoesäure-p-cyanophenylester
p-(Allyloxy-1-yloxy)-benzoesäure-p-cyano-m-fluorphenylester.

Beispiel 4

Durch Umsetzung äquimolarer Mengen trans-2-Butenylbromid mit 4-Jodphenol und Kaliumcarbonat in Dimethylformamid erhält man 4-(2-Buten-1-yl-oxy)-jodbenzol. Man löst 4,1 g dieser Verbindung und 2,6 g 4-n-Pentyl)-phenyl-acetylen [darstellbar aus 4-n-Pentylacetophenon durch Chlorierung mit Phosphorpentachlorid und anschließende Dehydrohalogenierung mittels Kalium-tert.-butylat] in 60 ml Triethylamin, fügt 0,2 g Bis-(triphenylphosphin)-palladium(II)-chlorid und 0,03 g Kupfer(I)-jodid hinzu und rührt 5 Stunden bei Raumtemperatur. Das Reaktionsgemisch wird mit Ether versetzt, über Kieselgel filtriert, zum Rückstand eingeengt und aus Ethanol umkristallisiert. Man erhält 4-(E-2-Buten-1-yl-oxy)-4′-n-pentyltolan.

Analog werden hergestellt:
4-(Allyloxy)-4′-ethyltolan
4-(Allyloxy)-4′-propyltolan
4-(Allyloxy)-4′-butyltolan
4-(Allyloxy)-4′-pentyltolan
4-(Allyloxy)-4′-heptyltolan
4-(2-Buten-1-yl-oxy)-4′-butyltolan
4-(2-Buten-1-yl-oxy)-4′-propyltolan
4-(2-Buten-1-yl-oxy)-4′-ethyltolan
4-(2-Buten-1-yl-oxy)-4′-heptyltolan
4-(2-Penten-1-yl-oxy)-4′-dodecyltolan
4-(2-Penten-1-yl-oxy)-4′-undexyltolan
4-(2-Penten-1-yl-oxy)-4′-decyltolan
4-(2-Penten-1-yl-oxy)-4′-nonyltolan
4-(2-Penten-1-yl-oxy)-4′-octyltolan
4-(2-Penten-1-yl-oxy)-4′-heptyltolan
4-(2-Penten-1-yl-oxy)-4′-hexyltolan
4-(2-Penten-1-yl-oxy)-4′-pentyltolan
4-(2-Penten-1-yl-oxy)-4′-butyltolan
4-(2-Penten-1-yl-oxy)-4′-propyltolan
4-(2-Penten-1-yl-oxy)-4′-ethyltolan
4-(2-Hexen-1-yl-oxy)-4′-decyloxytolan
4-(2-Hexen-1-yl-oxy)-4′-nonyloxytolan
4-(2-Hexen-1-yl-oxy)-4′-octyloxytolan
4-(2-Hexen-1-yl-oxy)-4′-heptyloxytolan
4-(2-Hexen-1-yl-oxy)-4′-hexyloxytolan
4-(2-Hexen-1-yl-oxy)-4′-pentyloxytolan
4-(2-Hexen-1-yl-oxy)-4′-butyloxytolan
4-(2-Hexen-1-yl-oxy)-4′-propyloxytolan
4-(2-Hexen-1-yl-oxy)-4′-ethoxytolan
4-(2-Hexen-1-yl-oxy)-4′-methoxytolan
4-(2-Hepten-1-yl-oxy)-4′-ethyltolan
4-(2-Hepten-1-yl-oxy)-4′-propyltolan
4-(2-Hepten-1-yl-oxy)-4′-butyltolan
4-(2-Hepten-1-yl-oxy)-4′-decyloxytolan
4-(2-Hepten-1-yl-oxy)-4′-nonyloxytolan
4-(2-Hepten-1-yl-oxy)-4′-octyloxytolan
4-(2-Hepten-1-yl-oxy)-4′-heptyloxytolan
4-(2-Hepten-1-yl-oxy)-4′-hexyloxytolan
4-(2-Hepten-1-yl-oxy)-4′-pentyloxytolan
4-(2-Hepten-1-yl-oxy)-4′-butyloxytolan
4-(2-Hepten-1-yl-oxy)-4′-propyloxytolan
4-(2-Hepten-1-yl-oxy)-4′-ethoxytolan
4-(2-Hepten-1-yl-oxy)-4′-methoxytolan
4-(2-Octen-1-yl-oxy)-4′-ethyltolan
4-(2-Octen-1-yl-oxy)-4′-propyltolan

4-(2-Octen-1-yl-oxy)-4'-butyltolan
4-(2-Octen-1-yl-oxy)-4'-decyloxytolan
4-(2-Octen-1-yl-oxy)-4'-nonyloxytolan
4-(2-Octen-1-yl-oxy)-4'-octyloxytolan
4-(2-Octen-1-yl-oxy)-4'-heptyloxytolan
4-(2-Octen-1-yl-oxy)-4'-hexyloxytolan
4-(2-Octen-1-yl-oxy)-4'-pentyloxytolan
4-(2-Octen-1-yl-oxy)-4'-butyloxytolan
4-(2-Octen-1-yl-oxy)-4'-propyloxytolan
4-(2-Octen-1-yl-oxy)-4'-ethoxytolan
4-(2-Octen-1-yl-oxy)-4'-methoxytolan
4-(2-Nonen-1-yl-oxy)-4'-ethyltolan
4-(2-Nonen-1-yl-oxy)-4'-propyltolan
4-(2-Nonen-1-yl-oxy)-4'-butyltolan
4-(2-Nonen-1-yl-oxy)-4'-dexyloxytolan
4-(2-Nonen-1-yl-oxy)-4'-nonyloxytolan
4-(2-Nonen-1-yl-oxy)-4'-octyloxytolan
4-(2-Nonen-1-yl-oxy)-4'-heptyloxytolan
4-(2-Nonen-1-yl-oxy)-4'-hexyloxytolan
4-(2-Nonen-1-yl-oxy)-4'-pentyloxytolan
4-(2-Nonen-1-yl-oxy)-4'-butyloxytolan
4-(2-Nonen-1-yl-oxy)-4'-propyloxytolan
4-(2-Nonen-1-yl-oxy)-4'-ethoxytolan
4-(2-Nonen-1-yl-oxy)-4'-methoxytolan

## Beispiel 5

Analog Chesterfield et al. (J. Chem. Soc. 4590 (1960)) wird 2-p-Bromphenyl-5-hydroxypyrimidin hergestellt. Man alkyliert dieses mit E-2-Hexen-1-yl-bromid in DMF in Gegenwart von $K_2CO_3$ und führt dann mit CuCN einen Brom-Nitril-Austausch durch: 33,1 g 2-p-Bromphenyl-5-(E-2-hexen-1-yloxy)-pyrimidin und 16,5 g CuCN werden unter Feuchtigkeitsausschluß und Stickstoffatmosphäre in 100 ml N-Methylpyrrolidon 8 Stunden lang auf 140°C erhitzt. Dann rührt man das noch nicht ganz erkaltete Reaktionsgemisch (ca. 100°C) in 300 ml Ammoniaklösung (halbkonzentriert) ein und extrahiert mit Toluol. Die Toluolphase wird noch mehrmals mit Ammoniaklösung und mit verdünntem HCl gewaschen, dann getrocknet und eingeengt. Der Rückstand wird durch Kristallisation und Chromatographie gereinigt. Man erhält 2-p-Cyanophenyl-5-(E-2-hexen-1-yloxy)-pyrimidin.

Analog werden hergestellt:

2-p-Cyanophenyl-5-(E-2-penten-1-yloxy)-pyrimidin
2-p-Cyanophenyl-5-(E-2-buten-1-yloxy)-pyrimidin
2-p-Cyanophenyl-5-(allyloxy)-pyrimidin
2-p-Cyano-m-fluorphenyl-5-(allyloxy)-pyrimidin.

## Beispiel 6

Zu 59,7 g 5 - [4 - (E - 2 - Hexenyl - 1 - oxy) - phenyl] - 2 - [4 - carbonyl-phenyl]-pyrimidin in 70 ml DMF gibt man nach G. A. Olah et al. Synthesis *1980*, 657 unter Stickstoffatmosphäre nach Feuchtigkeitsausschluß 9,8 g Cyanurchlorid und rührt dann das Reaktionsgemisch unter gelindem Erwärmen. Nach 12 Stunden wird wie üblich aufgearbeitet. Man erhält

5-[4-[E-2-Hexen-1-yloxy)-phenyl]-2-[4-cyanophenyl]-pyrimidin.

Das Ausgangsmaterial kann man nach folgendem Syntheseschema in Analogie zu bekannten Verfahren darstellen:

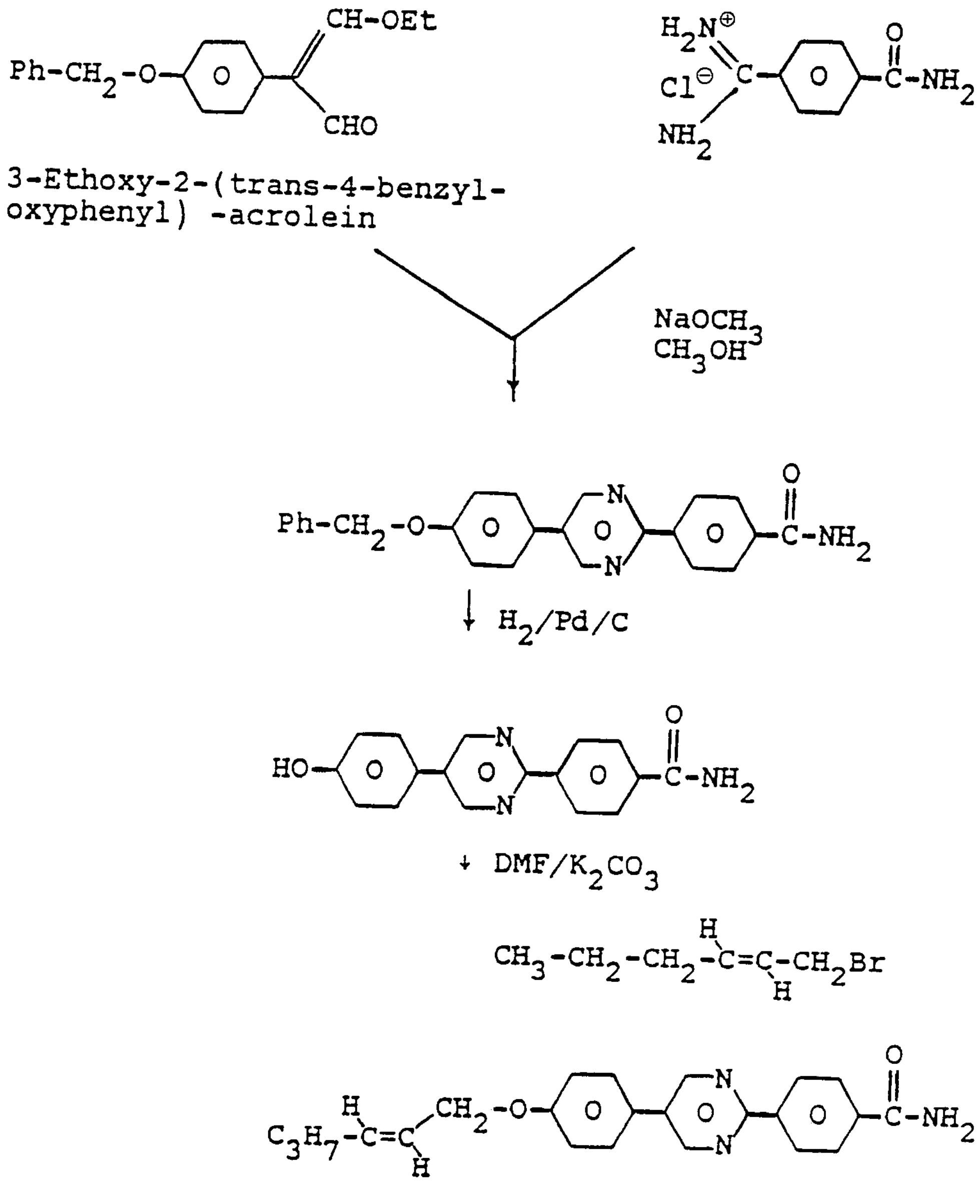


Analog wurden hergestellt:

5-[4-(E-2-Penten-1-yloxy)-phenyl]-2-[4-cyanophenyl]-pyrimidin
5-[4-(E-2-Buten-1-yloxy)-phenyl]-2-[4-cyanophenyl]-pyrimidin
5-[4-(Allyloxy)-phenyl]-2-[4-cyanophenyl]-pyrimidin
5-[trans-4-(E-2-Hexen-1-yloxy)-cyclohexyl]-2-[4-cyanophenyl]-pyrimidin
5-[trans-4-(E-2-Penten-1-yloxy)-cyclohexyl]-2-[4-cyanophenyl]-pyrimidin
5-[trans-4-(E-2-Buten-1-yloxy)-cyclohexyl]-2-[4-cyanophenyl]-pyrimidin
5-[trans-4-(Allyloxy)-cyclohexyl]-2-[4-cyanophenyl]-pyrimidin

Die nachfolgenden Beispiele betreffen erfindungsgemäße flüssigkristalline Phasen:

Beispiel A

Eine Flüssigkristallphase bestehend aus

12% trans,trans-4'-Allyloxycyclohexyl-4-propylcyclohexan
16% trans,trans-4'-Methoxycyclohexyl-4-pentylcyclohexan
10% trans,trans-4'-Ethoxycyclohexyl-4-pentylcyclohexan
10% trans,trans-4'-Propylcyclohexyl-4-butyryloxycyclohexan
3% trans,trans-4'-Propylcyclohexylcyclohexan-4'-carbonsäure-trans-4-propylcyclohexylester,
3% trans,trans-4-Propylcyclohexylcyclohexan-4'-carbonsäure-trans-4-pentylcyclohexylester,
3% trans,trans-4-Butylcyclohexylcyclohexan-4'-carbonsäure-trans-4-propylcyclohexylester,

3% trans,trans-4-Butylcyclohexylcyclohexyl-4'-carbonsäure-trans-pentylcyclohexylester,
3% 4,4'-Bis-(trans-4-propylcyclohexyl)-2-fluorbiphenyl,
3% 4,4'-Bis-(trans-4-pentylcyclohexyl)-2-fluorbiphenyl,
3% 4,4'-Bis-(trans-4-propylcyclohexyl)-biphenyl,
5% 4-(trans-4-Pentylcyclohexyl)-4'-(trans-4-propylcyclohexyl)-biphenyl,
4% 4-(trans-4-Pentylcyclohexyl)-4'-(trans-4-propylcyclohexyl)-2-fluorbiphenyl
15% p-trans-4-Propylcyclohexylbenzonitril und
7% trans-1-p-Ethoxyphenyl-4-propylcyclohexan

hat bis −40° keinen Phasenübergang smektisch/nematisch, eine dielektrische Anisotropie von +2,8, eine optische Anisotropie von 0,0830 und eine Schwellenspannung von 3,1 Volt.

**Patentansprüche für die Vertragsstaaten: CH DE GB**

1. Vinylen-Verbindungen der Formel I

$$R^1—(A^1—Z^1)_m—(A^2—Z^2)_n—(A^3)_o—O—(CH_2)_p—CH=CH—R^2 \qquad I$$

worin

$R^1$ Alkyl mit 1 bis 12 C-Atomen, worin auch eine oder zwei nicht benachbarte $CH_2$-Gruppen durch —O—, —O—CO—, —CO—O—, —CO— und/oder —CH=CH— ersetzt sein können, F, Cl, Br oder CN,

$R^2$ H oder Alkyl mit 1 bis 8 C-Atomen,

$A^1$, $A^2$ und $A^3$ jeweils unabhängig voneinander unsubstituiertes oder durch ein oder zwei F- und/oder Cl-Atome und/oder $CH_3$-Gruppen und/oder CN-Gruppen substituiertes 1,4-Phenylen, worin auch eine oder zwei CH-Gruppen durch N-Atome ersetzt sein können, unsubstituiertes oder durch ein oder zwei F- und/oder Cl-Atome und/oder $CH_3$-Gruppen und/oder CN-Gruppen substituiertes 1,4-Cyclohexylen, worin auch eine oder zwei nicht benachbarte $CH_2$-Gruppen durch O-Atome ersetzt sein können, 1,3-Dithian-2,5-diyl, 1,4-Bicyclo(2,2,2)octylen, Naphthalin-2,6-diyl, 1,2,3,4-Tetrahydronaphthalin-2,6-diyl, 1,2,3,4-Tetrahydronaphthalin-2,6-diyl oder trans-Dekahydronaphthalin-2,6-diyl,

$Z^1$ und $Z^2$ jeweils unabhängig voneinander —CO—O, —O—CO—, O—$CH_2$—, $CH_2$—O, —$CH_2CH_2$—, —CH=N—, —N=CH—, —C≡C— oder eine Einfachbindung,

m und o 1 oder 2,

n 0 oder 1, und

p 1 oder 2

bedeuten, wobei für m = 2 und/oder o = 2 die beiden Gruppen $A^1$ bzw. $A^3$ gleich oder voneinander verschieden sein können,

mit der Maßgabe, daß

a) falls n = 0, m = 1 und eine der Gruppen $A^1$ und $A^3$ eine 1,4-Phenylengruppe bedeutet, $Z^1$ nur —CO—O—, $OCH_2$—, —$CH_2$—O—, —$CH_2CH_2$— oder —C≡C— bedeutet, und

b) die Verbindungen
trans-4-Pentylcyclohexancarbonsäure-p-allyloxyphenylester
trans-4-(4-Pentenyl)cyclohexancarbonsäure-p-allyloxyphenylester
4-Allyloxy-1-[2-(trans-4-pentylcyclohexyl)ethyl]-benzol
4-(2E-Butenyloxy)-1-[2-(trans-4-propylcyclohexyl)-ethyl]-benzol
4-(3-Butenyloxy)-1-[2-(trans-4-propylcyclohexyl)-ethyl]-benzol
4-(3-Butenyloxy)-1-[2-(trans-4-pentylcyclohexyl)-ethyl]-benzol
4-Allyloxy-2-fluor-1-[2-(trans-4-pentylcyclohexyl)-ethyl]-benzol
4-(3-Butenyloxy)-2-fluor-1-[2-(trans-4-pentylcyclohexyl)-ethyl]-benzol

ausgenommen sind.

2. Vinylen-Verbindungen nach Anspruch 1 der Formel,

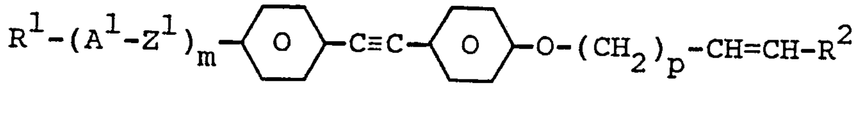

worin

$R^1$, $R^2$, $A^1$, $Z^1$ m und p die angegebene Bedeutung besitzen.

3. Vinylen-Verbindungen nach Anspruch 1 der Formel Iaa1,

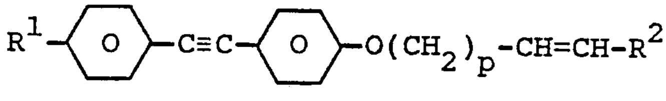

worin

$R^1$, $R^2$ und p die angegebene Bedeutung besitzen.

4. Verwendung der Verbindungen nach den Ansprüchen 1 bis 3 als Komponenten flüssigkristalliner Phasen.

5. Flüssigkristalline Phase mit mindestens 2 flüssigkristallinen Komponenten, dadurch gekennzeichnet, daß sie mindestens eine Verbindung nach den Ansprüchen 1 bis 3 enthält.

6. Flüssigkristall-Anzeigeelement, dadurch gekennzeichnet, daß es eine flüssigkristalline Phase nach Anspruch 5 enthält.

7. Elektrooptisches Anzeigeelement, dadurch gekennzeichnet, daß es als Dielektrikum eine flüssigkristalline Phase nach Anspruch 5 enthält.

**Patentansprüche für die Vertragsstaaten: AT BE NL**

1. Flüssigkristalline Phase mit mindestens zwei flüssigkristallinen Komponenten, dadurch gekennzeichnet, daß sie mindestens eine Vinylen-Verbindung der Formel I enthält,

$$R^1—(A^1—Z^1)_m—(A^2—Z^2)_n—(A^3)_o—O—(CH_2)_p—CH{=}CH—R^2 \qquad I$$

worin

$R^1$ Alkyl mit 1 bis 12 C-Atomen, worin auch eine oder zwei nicht benachbarte $CH_2$-Gruppen durch —O—, —O—CO—, —CO—O—, —CO— und/oder —CH=CH— ersetzt sein können, F, Cl, Br oder CN,

$R^2$ H oder Alkyl mit 1 bis 8 C-Atomen,

$A^1$, $A^2$ und $A^3$ jeweils unabhängig voneinander unsubstituiertes oder durch ein oder zwei F- und/oder Cl-Atome und/oder $CH_3$-Gruppen und/oder CN-Gruppen substituiertes 1,4-Phenylen, worin auch eine oder zwei CH-Gruppen durch N-Atome ersetzt sein können, unsubstituiertes oder durch ein oder zwei F- und/oder Cl-Atome und/oder $CH_3$-Gruppen und/oder CN-Gruppen substituiertes 1,4-Cyclohexylen, worin auch eine oder zwei nicht benachbarte $CH_2$-Gruppen durch O-Atome ersetzt sein können, 1,3-Dithian-2,5-diyl, 1,4-Bicyclo(2,2,2)octylen, Naphthalin-2,6-diyl, 1,2,3,4-Tetrahydronaphthalin-2,6-diyl, 1,2,3,4-Tetrahydronaphthalin-2,6-diyl oder trans-Dekahydronaphthalin-2,6-diyl,

$Z^1$ und $Z^2$ jeweils unabhängig voneinander —CO—O, —O—CO—, O—$CH_2$—, $CH_2$—O, —$CH_2CH_2$—, —CH=N—, —N=CH—, —C≡C— oder eine Einfachbindung,

m und o 1 oder 2,

n 0 oder 1, und

p 1 oder 2

bedeuten, wobei für m = 2 und/oder o = 2 die beiden Gruppen $A^1$ bzw. $A^3$ gleich oder voneinander verschieden sein können.

2. Flüssigkristalline Phase nach Anspruch 1, dadurch gekennzeichnet, daß sie mindestens eine Vinylen-Verbindung nach Anspruch 1 der Formel

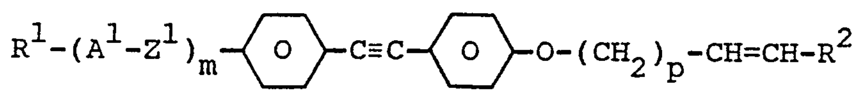

enthält,

worin

$R^1$, $R^2$, $A^1$, $Z^1$ m und p die angegebene Bedeutung besitzen.

3. Flüssigkristalline Phase nach Anspruch 1, dadurch gekennzeichnet, daß sie mindestens eine Vinylen-Verbindung nach Anspruch 1 der Formel Iaa1,

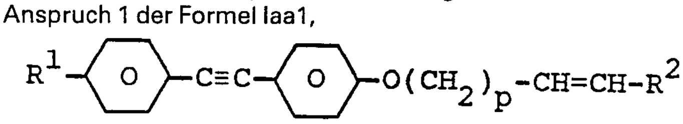

enthält,

worin

$R^1$, $R^2$ und p die angegebene Bedeutung besitzen.

4. Flüssigkristall-Anzeigeelement, dadurch gekennzeichnet, daß es eine flüssigkristalline Phase nach den Ansprüchen 1—3 enthält.

5. Elektrooptisches Anzeigeelement, dadurch gekennzeichnet, daß es als Dielektrikum eine flüssigkristalline Phase nach den Ansprüchen 1—3 enthält.

**Revendications pour les Etats contractants: CH DE GB**

1. Dérivés vinyléniques de formule I

$$R^1—(A^1—Z^1)_m—(A^2—Z^2)_n—(A^3)_o—O—(CH_2)_p—CH{=}CH—R^2 \qquad I$$

dans laquelle

$R^1$ représente un groupe alkyle en C1—C12 dans lequel un ou deux groupes $CH_2$ non voisins peuvent être remplacés par —O—, —O—CO—, —CO—O—, —CO— et/ou —CH=CH—; F, Cl, Br ou CN,

$R^2$ représente l'hydrogène ou un groupe alkyle en C1—C8,

$A^1$, $A^2$ et $A^3$ représentent chacun, indépendamment les uns des autres, un groupe 1,4-phénylène non

substitué ou substitué par un ou deux atomes de F et/ou de Cl et/ou groupes $CH_3$ et/ou groupes CN, et dans lequel un ou deux groupes CH peuvent être remplacés par des atomes de N, un groupe 1,4-cyclohexylène non substitué ou substitué par un ou deux atomes de F et/ou de Cl et/ou groupes $CH_3$ et/ou groupes CN, et dans lequel un ou deux groupes $CH_2$ non voisins peuvent être remplacés par des atomes de O, un groupe 1,3-dithianne-2,5-diyle, 1,4-bicyclo(2,2,2)octylène, naphtalène-2,6-diyle, 1,2,3,4-tétrahydronaphthalène-2,6-diyle, 1,2,3,4-tétrahydronaphtalène-2,6-diyle ou trans-décahydronaphtalène-2,6-diyle,

$Z^1$ et $Z^2$ représentent chacun, indépendamment l'un de l'autre, —CO—O—, —O—CO—, —O—$CH_2$—, —$CH_2$—O—, —$CH_2CH_2$—, —CH=N—, —N=CH—, —C≡C— ou une liaison simple, m et o sont égaux à 1 ou 2,

n est égal à 0 ou 1 et

p est égal à 1 ou 2,

étant précisé que, lorsque m = 2 et/ou que o = 2, les deux groupes $A^1$ et respectivement $A^3$ peuvent être identiques ou différents,

sous réserve que

a) lorsque n = 0, que m = 1 et que l'un des symboles $A^1$ et $A^2$ représente un groupe 1,4-phénylène, $Z^1$ ne peut représenter que —CO—O—, —$OCH_2$—, —$CH_2$—O—, —$CH_2CH_2$— ou —C≡C— et

b) les composés

trans-4-pentylcyclohexane-carboxylate de p-allyloxyphényle,
trans-4-(4-pentényl)-cyclohexane-carboxylate de p-allyloxyphényle,
2-allyloxy-1-[2-(trans-4-pentylcyclohexyl)-éthyl]benzène,
4-(2E-buténylоxy)-1-[2-(trans-4-propylcyclohexyl)-éthyl]-benzène,
4-(3-buténylоxy)-1-[2-trans-4-propylcyclohexyl)-éthyl]-benzène,
4-(3-buténylоxy)-1-[2-(trans-4-pentylcyclohexyl)-éthyl]-benzène,
4-allyloxy-2-fluoro-1-[2-(trans-4-pentylcyclohexyl)-éthyl]-benzène,
4-(3-buténylоxy)-2-fluoro-1-[2-(trans-4-pentylcyclohexyl)-éthyl]benzène

sont exclus.

2. Dérivés vinyléniques selon la revendication 1, de formule

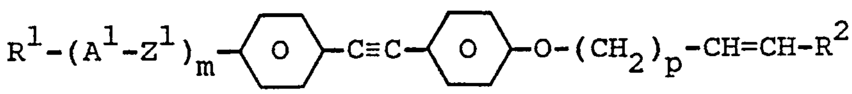

dans laquelle $R^1$, $R^2$, $A^1$, $Z^1$, m et p ont les significations indiquées ci-dessus.

3. Dérivés vinyléniques selon la revendication 1, de formule Iaa1,

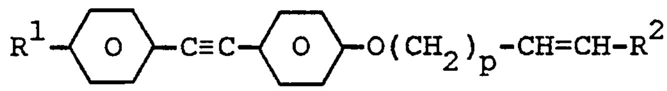

dans laquelle $R^1$, $R^2$ et p ont les significations indiquées ci-dessus.

4. Utilisation des composés selon les revendications 1 à 3 en tant que composants de phases à cristaux liquides.

5. Phase à cristaux liquides contenant au moins deux composants à cristaux liquides, caractérisée en ce qu'elle contient au moins un composé selon les revendications 1 à 3.

6. Elément d'affichage à cristaux liquides caractérisé en ce qu'il contient une phase à cristaux liquides selon la revendication 5.

7. Elément d'affichage électro-optique, caractérisé en ce qu'il contient en tant que diélectrique une phase à cristaux liquides selon la revendication 5.

**Revendications pour les Etats contractants: AT BE NL**

1. Phase à cristaux liquides à au moins deux composants à cristaux liquides, caractérisée en ce qu'elle contient au moins un dérivé vinylénique de formule I

$$R^1—(A^1—Z^1)_m—(A^2—Z^2)_n—(A^3)_o—O—(CH_2)_p—CH=CH—R^2 \qquad I$$

dans laquelle

$R^1$ représente un groupe alkyle en C1—C12 dans lequel un ou deux groupes $CH_2$ non voisins peuvent être remplacés par —O—, —O—CO—, —CO—O—, —CO— et/ou —CH=CH—; F, Cl, Br ou CN,

$R^2$ représente H ou un groupe alkyle en C1—C8,

$A^1$, $A^2$ et $A^3$ représentent chacun, indépendamment les uns des autres, un groupe 1,4-phénylène non substitué ou substitué par un ou deux atomes de F et/ou de Cl et/ou groupes $CH_3$ et/ou groupes CN, et dans lequel un ou deux groupes CH peuvent être remplacés par des atomes de N, un groupe 1,4-cyclohexylène non substitué ou substitué par un ou deux atomes de F et/ou de Cl et/ou groupes $CH_3$ et/ou groupes CN, et dans lequel un ou deux groupes $CH_2$ non voisins peuvent être remplacés par des atomes d'O, un groupe 1,3-dithianne-2,5-diyle, 1,4-bicyclo(2,2,2)octylène, naphtalène-2,6-diyle, 1,2,3,4-tétrahydronaphthalène-2,6-

diyle, 1,2,3,4-tétrahydronaphtalène-2,6-diyle ou trans-décahydronaphtalène-2,6-diyle,

$Z^1$ et $Z^2$ représentent chacun, indépendamment l'un de l'autre, —CO—O—, —O—CO—, —O—$CH_2$—, —$CH_2$—O—, —$CH_2CH_2$—, —CH=N—, —N=CH—, —C≡C— ou une liaison simple, m et o sont égaux à 1 ou 2,

n est égal à 0 ou 1 et

p est égal à 1 ou 2,

étant précisé que lorsque m = 2 et/ou que o = 2, les deux groupes $A^1$ et respectivement $A^3$ peuvent être identiques ou différents.

2. Phase à cristaux liquides selon la revendication 1, caractérisée en ce qu'elle contient au moins un dérivé vinylénique selon la revendication 1, de formule

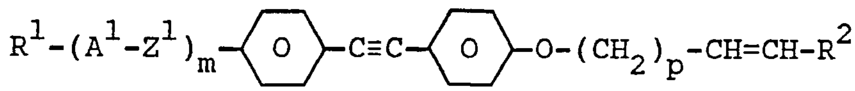

dans laquelle $R^1$, $R^2$, $A^1$, $Z^1$, m et p ont les significations indiquées ci-dessus.

3. Phase à cristaux liquides selon la revendication 1 caractérisée en ce qu'elle contient au moins un dérivé vinylénique selon la revendication 1, de formule Iaa1,

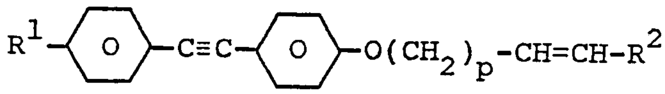

dans laquelle $R^1$, $R^2$ et p ont les significations indiquées ci-dessus.

4. Elément d'affichage à cristaux liquides, caractérisé en ce qu'il contient une phase à cristaux liquides selon les revendications 1 à 3.

5. Elément d'affichage électro-optique, caractérisé en ce qu'il contient en tant que diélectrique une phase à cristaux liquides selon les revendications 1 à 3.

**Claims for the contracting States: CH DE GB**

1. Vinylene compounds of the formula I

$$R^1—(A^1—Z^1)_m—(A^2—Z^2)_n—(A^3)_o—O—(CH_2)_p—CH=CH—R^2 \qquad I$$

wherein

$R^1$ is alkyl for 1 to 12 C atoms, it also being possible for one or two non-adjacent $CH_2$— groups to be replaced by —O—, —O—CO—, —CO—O—, —CO— and/or —CH=CH—, F, Cl, Br or CN,

$R^2$ is H or alkyl with 1 to 8 C atoms,

$A^1$, $A^2$, and $A^3$ are each independently of one another 1,4-phenylene which is unsubstituted or substituted by one or two F and/or Cl atoms and/or $CH_3$— groups and/or CN— groups, it also being possible for one or two CH— groups to be replaced by N atoms, or 1,4-cyclohexylene which is unsubstituted or substituted by one or two F and/or Cl atoms and/or $CH_3$— groups and/or CN— groups, it also being possible for one or two non-adjacent $CH_2$— groups to be replaced by O atoms, or 1,3-dithiane-2,5-diyl, 1,4-bicyclo(2,2,2)octylene, naphthalene-2,6-diyl, 1,2,3,4-tetrahydronaphthalene-2,6-diyl, 1,2,3,4-tetrahydronaphthalene-2,6-diyl (sic) or trans-decahydronaphthalene-2,6-diyl,

$Z^1$ and $Z^2$ are each independently of one another —CO—O—, —O—CO, —O—$CH_2$—, —$CH_2$—O—, $CH_2CH_2$—, —CH=N—, —N=CH—, —C≡C— or a single bond,

m and o are 1 or 2,

n is 0 or 1 and

p is 1 or 2

wherein, for m = 2 and/or o = 2, the two groups $A^1$ and $A^3$ can be identical or different from one another, with the proviso that

a) if n = 0, m = 1 and one of the groups $A^1$ and $A^3$ is a 1,4-phenylene group, $Z^1$ is only —CO—O—, $OCH_2$—, —$CH_2$—O—, —$CH_2CH_2$— or —C≡C—, and

b) the compounds

p-allyloxyphenyl trans-4-pentylcyclohexanecarboxylate

p-allyloxyphenyl trans-4-(pentenyl)cyclohexanecarboxylate

4-allyloxy-1-[2-(trans-4-pentylcyclohexyl)-ethyl]-benzene

4-(2E-butenyloxy)-1-[2-(trans-4-propylcyclohexyl)-ethyl]-benzene

4-(3-butenyloxy)-1-[2-(trans-4-propylcyclohexyl)-ethyl]-benzene

4-(3-butenyloxy)-1-[2-(trans-4-pentylcyclohexyl)-ethyl]-benzene

4-allyloxy-2-fluoro-1-[2-(trans-4-pentylcyclohexyl)-ethyl]-benzene and

4-(3-butenyloxy)-2-fluoro-1-[2-(trans-4-pentylcyclohexyl)-ethyl]-benzene

are excluded.

2. Vinylene compounds according to Claim 1, of the formula

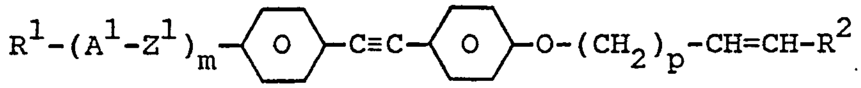

wherein

$R^1$, $R^2$, $A^1$, $Z^1$, m and p have the meaning given.

3. Vinylene compounds according to Claim 1 of the formula Iaa1

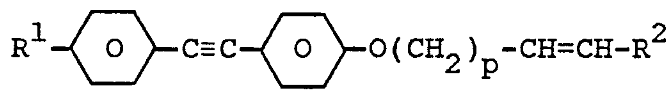

wherein

$R^1$, $R^2$ and p have the meaning given.

4. Use of the compounds according to Claims 1 to 3 as components of liquid crystal phases.

5. Liquid crystal phase with at least 2 liquid crystal components, characterized in that it contains at least one compound according to Claims 1 to 3.

6. Liquid crystal display element, characterized in that it contains a liquid crystal phase according to Claim 5.

7. Electrooptical display element, characterized in that it contains a liquid crystal phase according to Claim 5 as the dielectric.

**Claims for the contracting States: AT BE NL**

1. Liquid crystal phase with at least two liquid crystal components, characterized in that it contains at least one vinylene compound of the formula I

$$R^1—(A^1—Z^1)_m—(A^2—Z^2)_n—(A^3)_o—O—(CH_2)_p—CH=CH—R^2 \qquad I$$

wherein

$R^1$ is alkyl for 1 to 12 C atoms, it also being possible for one or two non-adjacent $CH_2$— groups to be replaced by —O—, —O—CO—, —CO—O—, —CO— and/or —CH=CH—, F, Cl, Br or CN,

$R^2$ is H or alkyl with 1 to 8 C atoms,

$A^1$, $A^2$, and $A^3$ are each independently of one another 1,4-phenylene which is unsubstituted or substituted by one or two F and/or Cl atoms and/or $CH_3$— groups and/or CN— groups, it is also being possible for one or two CH— groups to be replaced by N atoms, or 1,4-cyclohexylene which is unsubstituted or substituted by one or two F and/or Cl atoms and/or $CH_3$— groups and/or CN— groups, it also being possible for one or two non-adjacent $CH_2$— groups to be replaced by O atoms, or 1,3-dithiane-2,5-diyl, 1,4-bicyclo(2,2,2)octylene, naphthalene-2,6-diyl, 1,2,3,4-tetrahydronaphthalene-2,6-diyl, 1,2,3,4-tetrahydronaphthalene-2,6-diyl (sic) or trans-decahydronaphthalene-2,6-diyl,

$Z^1$ and $Z^2$ are each independently of one another —CO—O—, —O—CO, —O—$CH_2$—, —$CH_2$—O—, $CH_2CH_2$—, —CH=N—, —N=CH—, —C≡C— or a single bond,

m and o are 1 or 2,

n is 0 or 1 and

p is 1 or 2

wherein, for m = 2 and/or o = 2, the two groups $A^1$ and $A^3$ can be identical or different from one another.

2. Liquid crystal phase according to Claim 1, characterized in that it contains at least one vinylene compound according to Claim 1 of the formula

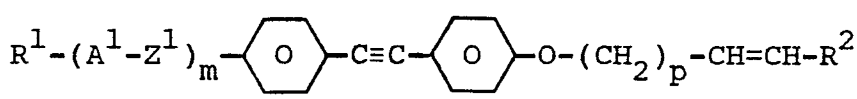

wherein

$R^1$, $R^2$, $A^1$, $Z^1$, m and p have the meaning given.

3. Liquid crystal phase according to Claim 1, characterized in that it contains at least one vinylene compound according to claim 1 of the formula IAA1

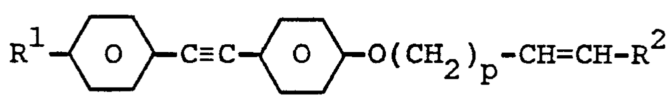

wherein

$R^1$, $R^2$ and p have the meaning given.

4. Liquid crystal display element, characterized in that it contains a liquid crystal phase according to Claims 1—3.

5. Electrooptical display element, characterized in that it contains a liquid crystal phase according to Claims 1—3 as the dielectric.